# FASCICULE DE BREVET EUROPEEN

(11) **EP 1 174 201 B1**
(45) Date de publication et mention de la délivrance du brevet: **25.01.2006**
(21) Numéro de dépôt: 01401883.2
(22) Date de dépôt: 13.07.2001
(51) Int. Cl.: B23B 1/00

(54) **Procédé de fabrication d'une denture sur une surface**
Verfahren zur Herstellung einer Verzahnung auf einer Oberfläche
Method for forming teeth on a surface

(30) Priorité: 20.07.2000 FR 0009527
(43) Date de publication de la demande: 23.01.2002
(73) Titulaire: Sferic Sarl, 41500 Menars (FR)
(72) Inventeur: Grimard, Jean-Christophe, 41120 Cellettes (FR); Cousin, Thierry, 41500 Menars (FR)
(74) Mandataire: Debay, Yves

(56) Documents cités:
- EP-A- 1 034 865
- DE-A- 19 849 872
- US-A- 4 427 872
- US-A- 5 665 091

## Description

La présente invention concerne un procédé d'usinage pour obtenir une denture sur la surface d'une forme quelconque.

En arthroplastie articulaire et quelle que soit l'articulation, il existe plusieurs types de prothèses qui diffèrent :
- selon la pathologie à traiter (traumatisme, arthrose, malformation,...),
- selon l'état de santé du patient,
- selon le type de voie d'abord et de technique opératoire (voie antérieure, voie postérieure, avec ou sans trochantérotomie...),
   pouvant engendrer des modifications au niveau :
- du type de géométrie de la prothèse (forme autobloquante ou non, anatomique ou droite, avec ou sans platine d'appui, section ovoïde ou rectangulaire,...),
- du type de fixation (avec ou sans ciment, avec ou sans revêtement,...)

Les chirurgiens souhaitent adapter la préparation du canal intra-médullaire de l'os, en fonction du type de prothèse qu'il souhaite ensuite disposer, afin d'assurer un geste opératoire efficace et sans risque, tout en garantissant la fiabilité de la fixation de l'implant.

La préparation médullaire ou calibrage de l'os est effectué à l'aide d'une râpe. Celle-ci façonne le canal intra-médullaire avec précision pour que le volume obtenu soit en correspondance avec le volume de l'implant à mettre en place. Pour cela, la râpe est couverte sur son enveloppe extérieure partiellement ou en totalité d'une denture qui va couper et tasser l'os spongieux et cortical.

Il existe à l'heure actuelle 3 familles de denture plus ou moins agressive :
- la denture droite,
- la denture droite avec brise-copeaux,
- la denture pointe diamant en forme de pyramide.

Ces différents types de denture sont plus ou moins bien adaptés au calibrage médullaire souhaité par le chirurgien en fonction du type de prothèse choisi.

Pour ces différents types de denture, il est possible de faire varier les paramètres des dents : angle de coupe, plat en sommet de dent, pas, orientation des dents.

De plus, pour les dents pyramidales, il est possible de faire varier l'angle d'inclinaison des faces de la pyramide. La pointe de la pyramide peut donc être orientée en opposition à la coupe.

Il est connu par le brevet US 5,665,091 une râpe pour préparer le canal intra-médullaire d'un os destiné à recevoir une prothèse. Cette râpe comprend un axe longitudinal et est constituée d'une face antérieure, d'une face postérieure et deux faces latérales, une face interne et une face externe, collaborant avec les parois internes du canal. Chaque face de la râpe est munie d'une denture différente pour préparer le canal. Certains types de dents, telles que celles appelées pointes de diamant, sont positionnés en rangées parallèles inclinées par rapport à l'axe longitudinal de la râpe. Les différentes dentures ont une efficacité différente, c'est-à-dire qu'elles sont plus ou moins agressives. Si les dents sont peu agressives, elles vont avoir un effet tassant dans l'os. En revanche, si elles sont très agressives, elles vont avoir un effet tranchant dans l'os. Les dents en pointes de diamant sont les plus agressives. Sur ce type de râpe, chaque type de dents est positionné sur une face de la râpe en fonction de la qualité osseuse de la zone de la paroi interne du canal intra-méduliaire avec laquelle cette face de la râpe est en contact.

Le problème de ce type de râpe réside dans le fait que la répartition des dents sur la surface n'est pas homogène. En effet, les procédés permettant d'élaborer les différents types de dents sont limités et souvent ils ne permettent pas de générer certains types de dents dans certaines zones de la râpe ou alors la forme des dents, dans ces zones, n'est plus maîtrisée, par exemple, à la jonction de deux faces. Ainsi, l'efficacité et la précision du calibrage ne sont pas garanties et le risque de rupture de la râpe, par concentration des contraintes en service, est important.

La présente invention a donc pour objet de pallier les inconvénients de l'art antérieur en proposant un procédé d'usinage continu adapté à une forme quelconque, permettant de maîtriser les différents types de denture et les caractéristiques géométriques des dents.

Un but de l'invention est de proposer un procédé d'usinage dans lequel les contraintes mécaniques en service sont réparties sur toute la surface de la forme et où il est possible d'obtenir une répartition des dents homogène.

Ce but est atteint par l'utilisation du procédé selon la revendication 1.

Des développements supplémentaires de l'invention sont décrits dans les revendications 2 à 14.

L'invention, avec ses caractéristiques et avantages, ressortira plus clairement à la lecture de la description faite en référence aux dessins annexés
dans lesquels :
- la figure 1 représente, en vue de côté, la râpe sur laquelle sont tracées quelques génératrices,
- la figure 2 représente, en vue suivant F, la face supérieure de la râpe,
- la figure 3 représente une portion de la râpe en perspective sur laquelle est tracée une portion de courbe en hélice,
- la figure 4 représente, en vue de côté et simplifiée, la râpe avant usinage, sur laquelle a été tracée une courbe en forme d'hélice,
- la figure 5 représente, en vue de côté, l'outil utilisé pour l'usinage,
- la figure 6 représente, en perspective, le corps d'une râpe usiné partiellement suivant une hélice de pas constant,
- la figure 7 représente, en vue de côté et simplifiée, la râpe à la suite d'un usinage,
- la figure 8 représente, en vue de côté et simplifiée, la râpe sur laquelle a été tracée deux courbes en hélice,
- la figure 9 représente, en vue de côté et simplifiée, la râpe après un usinage suivant deux courbes en hélice,
- la figure 10 représente une section de la râpe suivant l'inclinaison d'une courbe en hélice après un usinage suivant deux courbes en hélice,
- la figure 11 représente, en vue de côté, les dents obtenues après l'usinage suivant les deux hélices,
- la figure 12 représente, en perspective, une dent en forme de pyramide régulière.

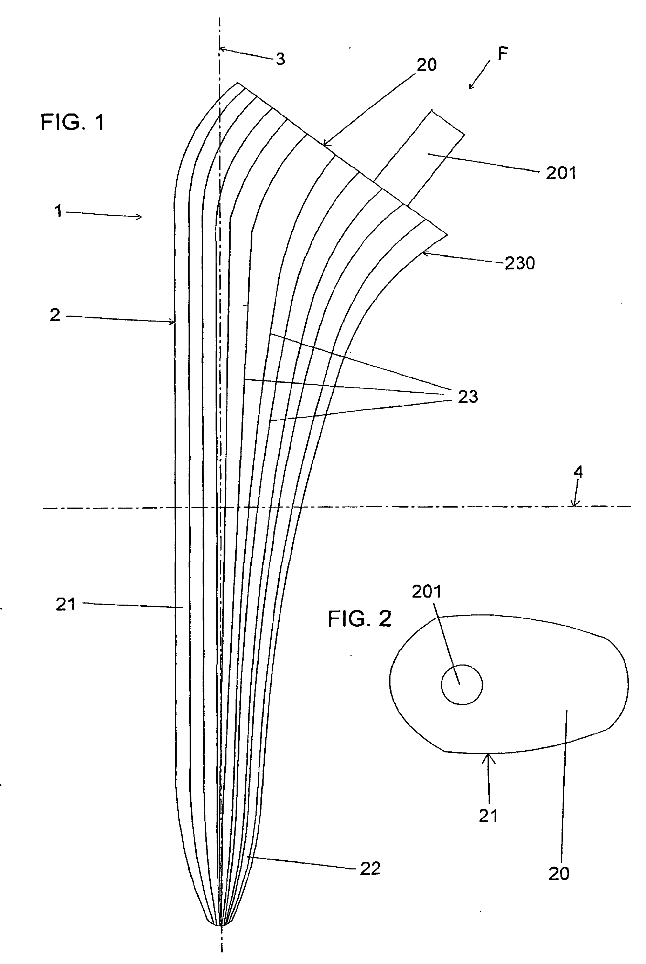

La râpe sera d'abord décrite en liaison avec les figures 1 à 7.

Une râpe (1) est destinée à préparer le canal intra-médullaire d'un os dans lequel est insérée une prothèse. La râpe (1) présentée est appropriée pour calibrer le canal du fémur. Chaque râpe (1) a une forme variable selon la forme du canal intra-médullaire de l'os que l'utilisateur souhaite préparer.

Selon une forme connue, la râpe est constituée d'un corps (2) comprenant une face supérieure (20) et une enveloppe extérieure (21) chargée de râper la paroi interne du canal. Le corps (2) se termine par une forme profilée (22) destinée à faciliter l'enfoncement de la râpe (1) dans le canal. Des éléments géométriques, tels que des tourillons (201), des rainures ou des alésages, mâles ou femelles, disposés sur la face supérieure (20) du corps (2) constituent un système d'encliquetage destiné à recevoir un embout constituant l'extrémité d'un manche pour manipuler la râpe dans le canal et destiné à recevoir éventuellement des composants prothétiques d'essai. La face supérieure (20) du corps (2) peut être plane ou peut comporter un tenon permettant la soudure d'un manche pour manipuler la râpe (1) dans l'os.

Le corps (2) de la râpe (1) a une forme allongée selon un axe (3) sensiblement parallèle à l'axe longitudinal du canal. La section transversale du corps (2) de la râpe (1) est variable le long du corps (2), puis décroissante sur son extrémité finale, pour se terminer par la forme en profilé (22) destinée à faciliter l'enfoncement de la râpe (1). La forme de la section transversale de la partie inférieure de la râpe (1) est sensiblement constante. Sur la partie supérieure du corps (2) de la râpe (1), la forme de la section transversale du corps (2) est progressivement variable et supérieure à la section transversale de la partie inférieure du corps (2). La jonction entre la partie inférieure et la partie supérieure se fait par une continuité des courbes formant les surfaces. La face supérieure (20) du corps (2) est inclinée par rapport à l'axe (3) de la râpe (1).

Selon une première variante, un usinage suivant une courbe géométrique ayant la forme d'une hélice (210) est effectué sur la paroi extérieure (21) du corps (2) de la râpe (1).

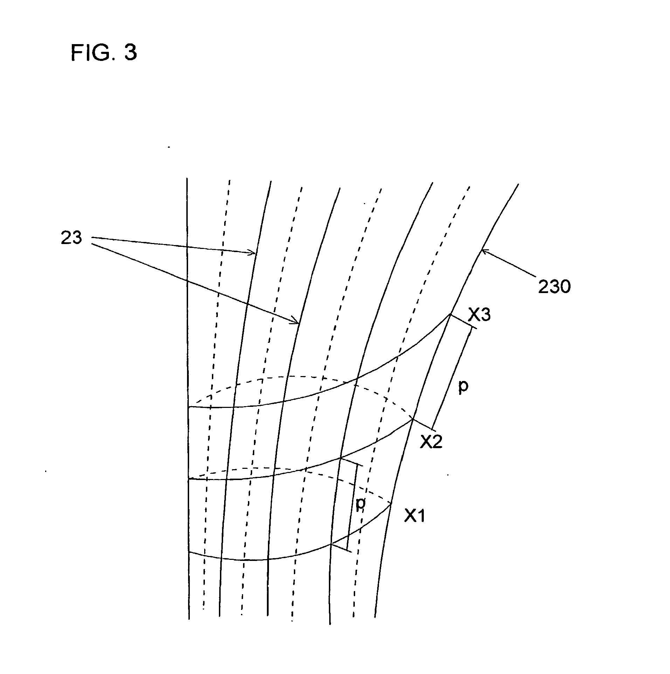

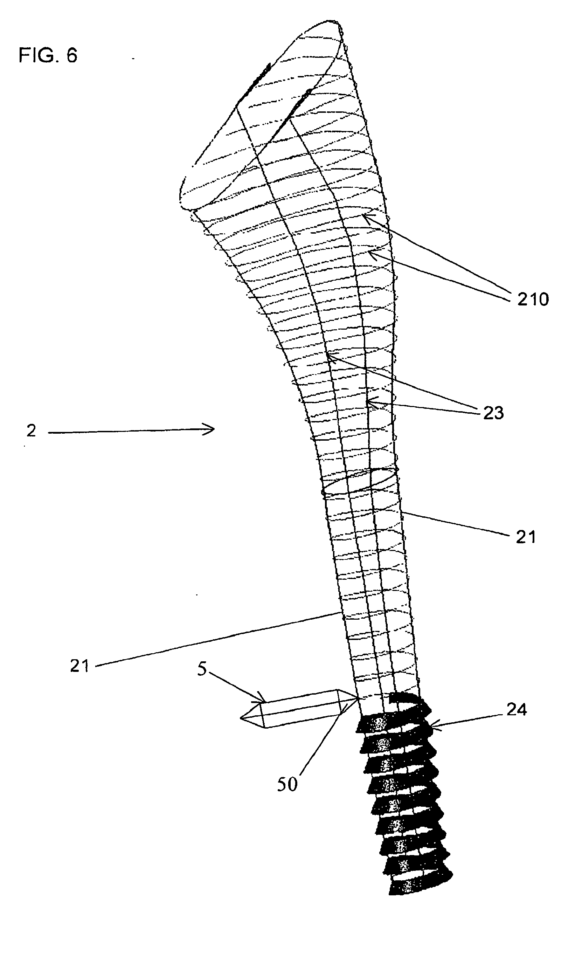

Pour cela, la courbe en hélice (210) est d'abord dessinée sur l'enveloppe extérieure (21) du corps (2) de la râpe (1) à l'aide d'un logiciel informatique. Ce logiciel permet de construire des hélices à pas constant ou variable sur une forme quelconque provenant d'un fichier. Le logiciel utilise la méthode suivante pour construire une hélice sur une forme quelconque, telle que celle de la râpe (1) de l'invention. La forme du corps (2) de la râpe est définie par des génératrices (23) s'appuyant sur la surface enveloppe (21) comme représenté figures 1 et 3. Sur une génératrice (230) du corps (2) de la râpe (1), plusieurs points sont positionnés, distants entre chacun d'eux du pas constant, défini par p, de la courbe en hélice que l'on souhaite tracer. Sur la figure 3, à partir d'un de ces points, défini par X1, une portion d'hélice est tracée sur une révolution complète de la forme de la râpe, la portion d'hélice joignant le point de la génératrice adjacent de X1, par exemple supérieur, défini par X2. Dans la continuité de la première portion d'hélice est tracée une seconde portion d'hélice joignant X2 au point de la génératrice adjacent supérieur de X2, défini par X3. Cette opération est reproduite avec une troisième portion d'hélice et ainsi de suite, de manière à ce que toute l'enveloppe (21) soit parcourue par une courbe en hélice (210) continue de pas constant. Nous obtenons ainsi une râpe sur laquelle est tracée une courbe en hélice (210), comme représenté sur la figure 6. Dans une variante, on peut imaginer un pas variable d'hélice le long du corps (2) de la râpe (1).

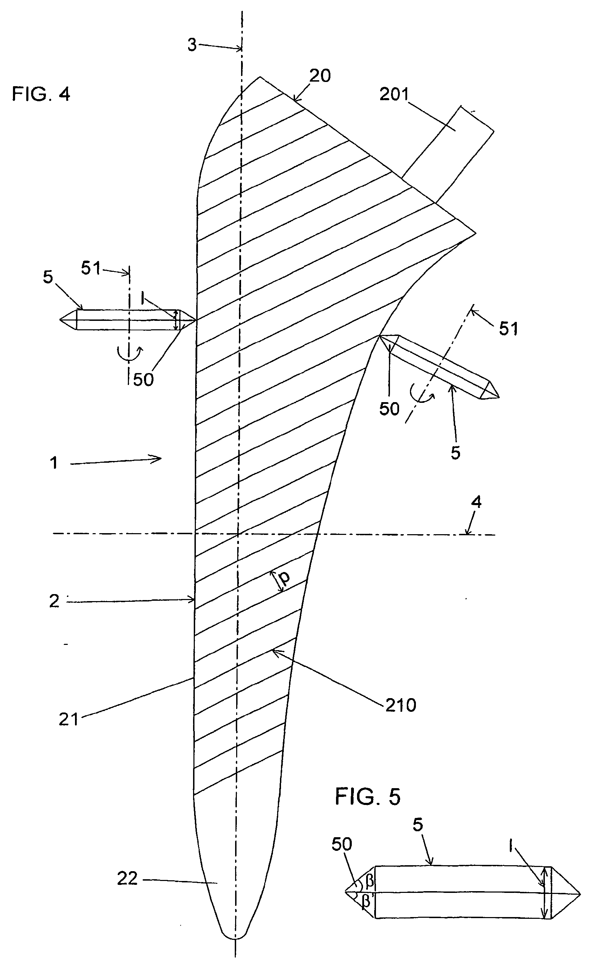

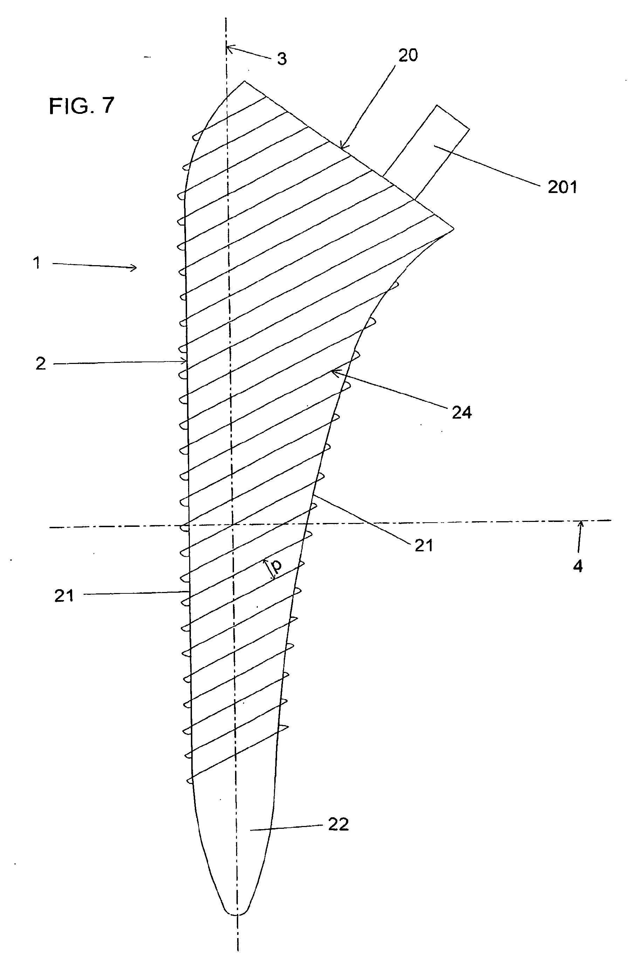

La figure 4 est une représentation simplifiée de la râpe sur laquelle est tracée une courbe en hélice de pas constant. En effet, le pas de la courbe en hélice que l'on souhaite représenter étant constant et la section transversale du corps (2) de la râpe (1) étant variable, l'angle de la courbe hélice, défini par rapport à l'axe (4) perpendiculaire à l'axe (3) de la râpe (1), n'est en réalité pas constant comme cela est représenté sur cette figure. La section transversale dans la partie supérieure du corps (2) de la râpe (1) étant supérieure à celle de la partie inférieure du corps (2) de la râpe (1), l'angle de la courbe en hélice varie en décroissant du bas vers le haut du corps (2) de la râpe (1). Les représentations sont également simplifiées sur les figures 7, 8 et 9.

Dans le cas d'un pas constant, l'angle de la courbe en hélice est variable le long du corps (2) de la râpe (1), mais on parlera tout de même d'inclinaison d'hélice correspondant à l'angle maximum de la courbe en hélice. Cet angle est maximum là où la section transversale du corps (2) de la râpe (1) est la plus petite, c'est à dire sur la partie inférieure du corps (2) de la râpe (1).

Une fois l'hélice (210) dessinée, l'usinage peut débuter suivant le tracé de la courbe en hélice, comme représenté figure 6. L'usinage est réalisé avec un outil (5) représenté figure 5, fraise ou meule, ayant une tête (50) d'attaque de forme biseautée. Cette tête (50) peut avoir des largeurs I différentes et un biseau dont l'angle, défini par β et β' égaux ou différents, peut varier suivant l'efficacité que l'on souhaite donner à la râpe (1). La profondeur d'usinage varie en fonction de la valeur de la largeur 1 et de l'angle de la tête biseautée. La fraise ou meule, est entraînée en rotation selon un axe (51) maintenu en permanence parallèle aux tangentes aux points appartenant aux génératrices (23) successives et formant la courbe en hélice (210), comme représenté figures 4 et 6.

Dans une première étape, en chaque point de l'hélice (210), le logiciel trace la normale à la surface de l'enveloppe extérieure (21) du corps (2). Ensuite, l'outil (5) usine l'enveloppe extérieure (21) du corps (2) de la râpe (1) suivant l'hélice (210) tracée, la tête (50) de l'outil (5) étant orientée de manière à ce qu'elle suive l'orientation des normales tracées. Cet usinage est réalisé sur une machine comportant au minimum cinq axes.

Une fois l'usinage suivant l'hélice (210) terminé, le corps (2) de la râpe (1) présente un filet (24), comme représenté figures 6 et 7. Ce profil en filet (24) permet de limiter les risques de rupture, car les efforts de travail ne sont plus concentrés uniquement sur la section la plus faible mais ils se répartissent sur toute la hauteur de la denture. De plus, l'usinage continu en hélice permet d'éviter les surpressions de calibrage au niveau du proche environnement physiologique limitant ainsi le risque d'embolie graisseuse lors de l'utilisation de la râpe, car la zone de dégagement du copeau osseux n'est plus fermée.

Dans le cas où l'usinage est réalisé suivant une hélice (210) d'inclinaison faible, la râpe dispose alors d'une denture du type denture droite.

La largeur de la tête (50) de l'outil (5) en forme de biseau, définie par 1, pourra, par exemple, être sensiblement égale ou supérieure au pas p de l'hélice (210), de manière à former un filet (24) coupant et donc plus efficace.

Le pas de l'hélice (210) pourra, par exemple, être variable le long du corps (2) de la râpe (1).

II est possible d'usiner la râpe suivant plusieurs hélices d'inclinaisons différentes et décalées le long de l'axe (3) de la râpe (1). Dans ce cas, l'usinage peut être réalisé à une profondeur différente suivant chaque hélice pour former une denture dite avec brise-copeaux.

Selon une deuxième variante, l'usinage est réalisé suivant deux courbes en hélice (211, 212) sur l'enveloppe extérieure (21) du corps (2) de la râpe (1).

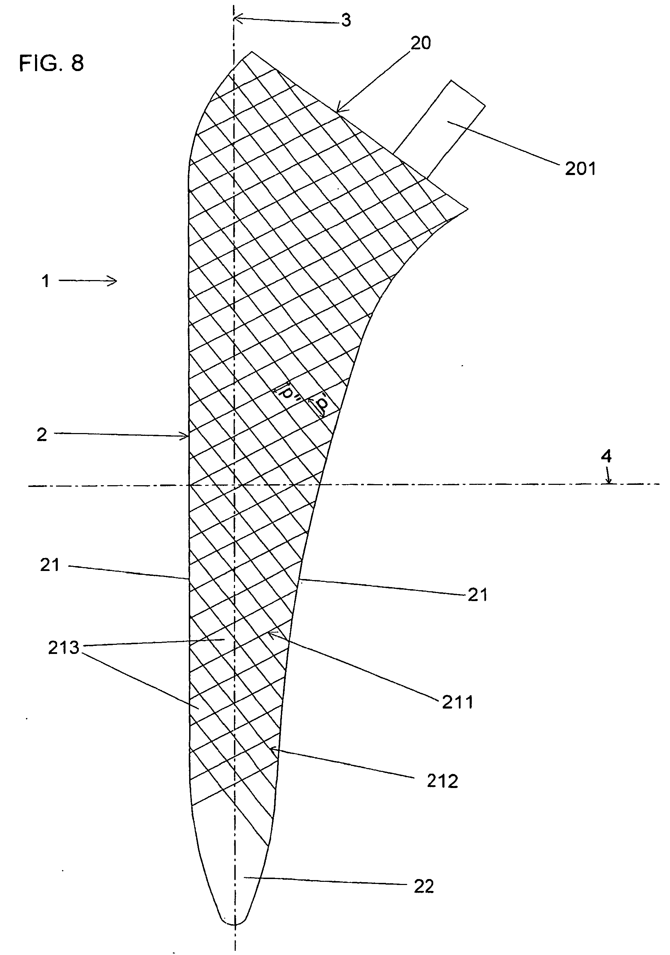

L'invention sera maintenant décrite en liaison avec les figures 8 à 12.

Deux courbes en hélice (211, 212) sont d'abord tracées selon la méthode présentée plus haut, comme représenté figure 8. Ces hélices (211, 212) ont la particularité d'être de pas inversés. Elles seront préférentiellement d'inclinaisons par rapport à l'axe (4) différentes. Les deux hélices (211, 212) peuvent avoir des pas constants, respectivement p' et p", égaux ou différents. Les espaces (213) ainsi formés par les intersections des deux hélices (211, 212) sont réguliers et répartis de manière homogène sur toute la surface du corps (2) de la râpe (1).

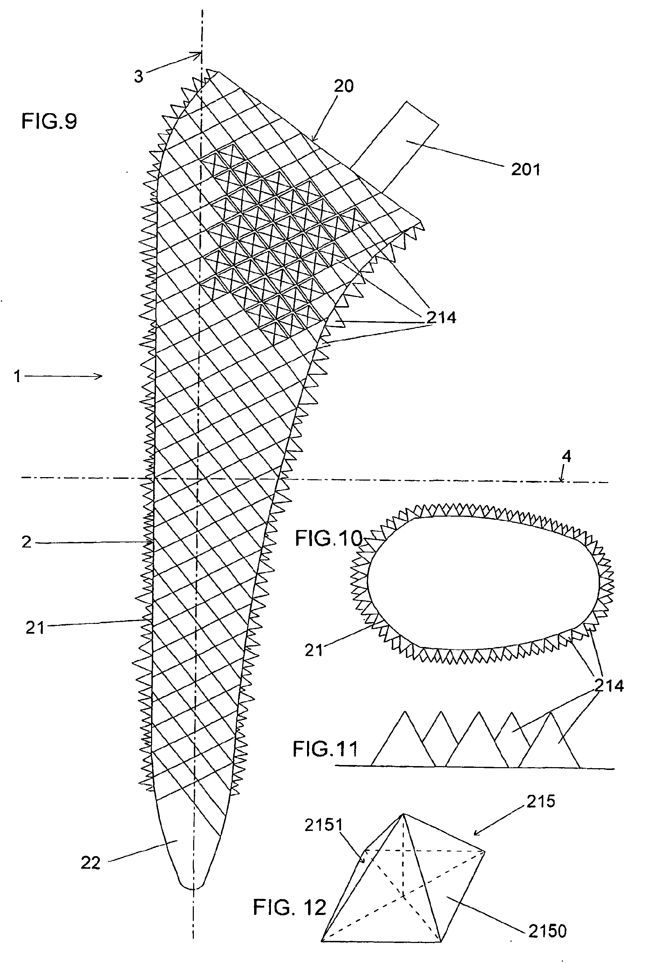

Les deux usinages sont réalisés selon la méthode présentée plus haut. La tête (50) de l'outil (5) est choisie de manière à ce que sa largeur 1 soit sensiblement égale ou supérieure au pas (p', p") de l'hélice (211, 212) qu'il suit, pour former, après usinage, un filet coupant. L'usinage avec cet outil (5) suivant les deux hélices (211, 212) de pas inversés va donc former des dents (214), comme représenté figure 8. Ces dents (214), de par la forme en biseau de la tête (50) de l'outil (5), avec β et β' égaux et dans le cas d'une profondeur de pénétration de l'outil (5) constante et égale suivant les deux hélices (211, 212), de pas p' et p" égaux, sont des pointes en forme de pyramides régulières (215), comme représenté figures 11 et 12. Ces pyramides (215) ont pour base (2150) les espaces (213) formés par les intersections des deux hélices (211, 212) de pas inversés et leur hauteur est normale à la surface du corps (2) de la râpe (1). Ces dents (214) en forme de pyramides régulières (215) sont des pointes de diamant et sont très agressives. L'angle de la tête (50) en biseau défini par β et β' pourra, par exemple, être de 90° de manière à former des pyramides (215) dont les faces latérales (2151) font un angle de 45° avec la base.

Il est possible de réaliser des pyramides plates ou rayonnées sur leur partie supérieure en incluant dans le profil de la tête (50) biseautée de l'outil (5) une partie plane ou rayonnée.

Dans le cas où les inclinaisons des hélices inversées sont choisies différentes, cela permet d'éviter que les dents (214) pyramidales formées soient alignées suivant l'axe (3) de la râpe (1). En décalant la position des dents (214) placées les unes au-dessus des autres, un calibrage complet de toute la paroi intra-médullaire en correspondance avec l'implant est réalisé, en évitant la formation de stries verticales dans l'os.

Il peut être choisi d'incliner la tête (50) de l'outil (5) par rapport à sa position normale à la surface, de manière à former des pyramides irrégulières sur certaines parties de la râpe. De même, l'angle de la tête (50) en biseau peut varier d'un usinage à l'autre et en jouant sur la valeur des angles β et β' de la tête en biseau, c'est à dire avec β différent de β', il est possible d'obtenir des pyramides irrégulières dont le sommet est décalé.

Selon une autre variante, les pas p' et p" de chaque hélice (211, 212) ne sont pas constants et varient le long du corps (2) de la râpe (1). Dans ce dernier cas, la section (213) constituant la base des dents (214) sera variable en fonction de l'évolution des pas p' et p".

Selon une autre variante, les deux profondeurs d'usinage suivant respectivement les deux hélices (211, 212) sont différentes, de manière à créer une denture avec brise-copeaux.

Cet usinage suivant deux hélices de pas inversés, permet donc d'obtenir une répartition homogène des dents (214) sur toute l'enveloppe extérieure (21) du corps (2) de la râpe (1) et en particulier aux endroits de jonction plus arrondis de l'enveloppe (21). Ainsi, le calibrage de la paroi interne du canal intra-médullaire sera complet et homogène, toute la surface en correspondance avec l'implant aura été travaillée.

Sur la figure 9, la hauteur des dents est représentée irrégulière car les dents (214) les plus hautes sont celles du premier plan et les plus basses celles de l'arrière plan. Il faut bien comprendre que, par rapport à un contour de râpe (1) correspondant à une section suivant l'inclinaison d'une hélice (211, 212) comme représenté à la figure 9, la hauteur des dents (214) est constante sur ce contour dans le cas où les deux pas p' et p" sont constants et la pénétration de l'outil (5) également.

Une râpe usinée suivant deux hélices aura, par exemple les caractéristiques suivantes. La râpe pourra être en acier inoxydable X5 Cr Ni Cu Nb 16.04 traité thermiquement. Elle pourra avoir une longueur L de 150 mm, une section D de 12 mm, un pas p' de 3 mm, un pas p" de 3 mm également, des angles α' et α" respectivement de 30° et de 50° et un rayon de fond de dent de 0,5 mm.

Ainsi, le procédé d'usinage pour obtenir une denture sur la surface d'une forme quelconque par un outil (5) muni d'une tête (50), suivant au moins une courbe géométrique tracée sur l'enveloppe de la forme quelconque et ayant la forme d'une hélice (210), est caractérisé en ce qu'il comprend les étapes suivantes :
- tracer la normale à la surface en une pluralité de points pris le long de l'hélice (210),
- faire suivre l'hélice à la tête (50) de l'outil (5), en orientant la tête (5) de manière à ce qu'elle suive l'orientation des normales déterminées.

Selon une autre particularité, l'usinage est réalisé avec un outil (5) disposant d'une tête (50) de forme biseautée.

Selon une autre particularité, l'usinage est réalisé suivant une hélice (210) de faible inclinaison, de manière à obtenir une denture d'aspect type denture droite.

Selon une autre particularité, l'usinage est réalisé à des profondeurs différentes respectivement suivant plusieurs hélices d'inclinaisons différentes, de manière à obtenir une denture avec brise-copeaux.

Selon une autre particularité, l'usinage est réalisé suivant deux hélices (211, 212) de pas inversés, de manière à obtenir des dents (214), les dents (214) ayant pour base (213) les espaces formés par les intersections des deux hélices (211, 212).

Selon une autre particularité, l'usinage est réalisé suivant les deux hélices (211, 212) à la même profondeur, de manière à obtenir une denture type pointe de diamant, l'orientation des dents étant normale à l'enveloppe.

Selon une autre particularité, les pas des deux hélices (211, 212) sont constants et égaux, de manière à obtenir des dents (214) ayant la forme de pyramides régulières (215).

Selon une autre particularité, les deux hélices (212, 213) ont des inclinaisons différentes de manière à ce que les dents (214) formées par le passage de l'outil (5) ne soient pas alignées.

Selon une autre particularité, le pas de chaque hélice (210, 211, 212) est constant le long de l'enveloppe de la forme quelconque.

Selon une autre particularité, les dents (214) peuvent être plates ou rayonnées sur leur partie supérieure, en incluant dans le profil de la tête (50) biseautée de l'outil (5) une partie plane ou rayonnée.

La râpe (1) obtenue en utilisant le procédé présenté ci-dessus et destinée à préparer le canal intra-médullaire d'un os pour que celui-ci reçoive une prothèse, est caractérisée en ce qu'elle comprend un corps (2), allongé selon un axe (3) sensiblement parallèle à l'axe longitudinal du canal, à section transversale variable, constitué d'une face dite supérieure (20) et d'une enveloppe extérieure (21), et a une forme apte à collaborer avec la paroi interne du canal intra-méduilaire de l'os.

Selon une autre particularité, le corps (2) de la râpe (1) se termine par une forme profilée (22) pour faciliter l'enfoncement de la râpe (1) dans le canal.

Selon une autre particularité, des éléments géométriques mâles ou femelles, disposés sur la face supérieure (20) du corps (2) constituent un système d'encliquetage destiné à recevoir un embout constituant l'extrémité d'un manche pour manipuler la râpe (1) dans le canal et destiné à recevoir éventuellement des composants prothétiques d'essais.

Selon une autre particularité, la face supérieure (20) du corps (2) est plane ou comporte un tenon permettant la soudure d'un manche pour manipuler la râpe (1) dans l'os.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Procédé d'usinage pour obtenir une denture sur la surface d'une forme quelconque par un outil (5) muni d'une tête (50), suivant au moins une courbe géométrique tracée sur l'enveloppe de la forme quelconque et ayant la forme d'une hélice (210), **caractérisé en ce qu'**il comprend les étapes suivantes :
- tracer la normale à la surface en une pluralité de points pris le long de l'hélice (210),
- faire suivre l'hélice à la tête (50) de l'outil (5), en orientant la tête (5) de manière à ce qu'elle suive l'orientation des normales déterminées.

2. Procédé d'usinage pour obtenir une denture sur la surface d'une forme quelconque selon la revendication 1, **caractérisée en ce que** l'usinage est réalisé avec un outil (5) disposant d'une tête (50) de forme biseautée.

3. Procédé d'usinage pour obtenir une denture sur la surface d'une forme quelconque selon la revendication 2, **caractérisé en ce que** l'usinage est réalisé suivant une hélice (210) de faible inclinaison, de manière à obtenir une denture d'aspect type denture droite.

4. Procédé d'usinage pour obtenir une denture sur la surface d'une forme quelconque selon la revendication 2, **caractérisée en ce que** l'usinage est réalisé à des profondeurs différentes respectivement suivant plusieurs hélices d'inclinaisons différentes, de manière à obtenir une denture avec brise-copeaux.

5. Procédé d'usinage pour obtenir une denture sur la surface d'une forme quelconque selon la revendication 2, **caractérisée en ce que** l'usinage est réalisé suivant deux hélices (211, 212) de pas inversés, de manière à obtenir des dents (214), les dents (214) ayant pour base (213) les espaces formés par les intersections des deux hélices (211, 212).

6. Procédé d'usinage pour obtenir une denture sur la surface d'une forme quelconque selon la revendication 5, **caractérisé en ce que** l'usinage est réalisé suivant les deux hélices (211, 212) à la même profondeur, de manière à obtenir une denture type pointe de diamant, l'orientation des dents étant normale à l'enveloppe.

7. Procédé d'usinage pour obtenir une denture sur la surface d'une forme quelconque selon la revendication 6, **caractérisé en ce que** les pas des deux hélices (211, 212) sont constants et égaux, de manière à obtenir des dents (214) ayant la forme de pyramides régulières (215).

8. Procédé d'usinage pour obtenir une denture sur la surface d'une forme quelconque selon l'une quelconque des revendications 5 à 7, **caractérisé en ce que** les deux hélices (212, 213) ont des inclinaisons différentes de manière à ce que les dents (214) formées par le passage de l'outil (5) ne soient pas alignées.

9. Procédé d'usinage pour obtenir une denture sur la surface d'une forme quelconque selon l'une quelconque des revendications 1 à 6 et 8, **caractérisé en ce que** le pas de chaque hélice (210, 211, 212) est constant le long de l'enveloppe de la forme quelconque.

10. Procédé d'usinage pour obtenir une denture sur la surfacé d'une forme quelconque selon la revendication 5, **caractérisé en ce que** les dents (214) peuvent être plates ou rayonnées sur leur partie supérieure, en incluant dans le profil de la tête (50) biseautée de l'outil (5) une partie plane ou rayonnée.

11. Utilisation du procédé selon les revendications 1 à 10 pour former une râpe (1) destinée à préparer le canal intra-médullaire d'un os pour que celui-ci reçoive une prothèse, **caractérisé en ce que** la râpe (1) comprend un corps (2), allongé selon un axe (3) sensiblement parallèle à l'axe longitudinal du canal, à section transversale variable, constitué d'une face dite supérieure (20) et d'une enveloppe extérieure (21), et a une forme apte à collaborer avec la paroi interne du canal intra-médullaire de l'os.

12. Utilisation du procédé selon la revendication 11, **caractérisée en ce que** le corps (2) de la râpe (1) se termine par une forme profilée (22) pour faciliter l'enfoncement de la râpe (1) dans le canal.

13. Utilisation du procédé selon la revendication 11 ou 12, **caractérisé en ce que** des éléments géométriques mâles ou femelles, disposés sur la face supérieure (20) du corps (2) constituent un système d'encliquetage destiné à recevoir un embout constituant l'extrémité d'un manche pour manipuler la râpe (1) dans le canal et destiné à recevoir éventuellement des composants prothétiques d'essais.

14. Utilisation du procédé selon l'une quelconque des revendications 11 à 13, **caractérisé en ce que** la face supérieure (20) du corps (2) est plane ou comporte un tenon permettant la soudure d'un manche pour manipuler la râpe (1) dans l'os.

## Patentansprüche

1. Bearbeitungsverfahren zum Erzielen einer Verzahnung an der Oberfläche einer beliebigen Form entlang mindestens einer geometrischen Kurve, welche an der Hülle der beliebigen Form aufgetragen ist und die Form einer Spirale (210) aufweist, mit Hilfe eines Werkzeugs (5), das mit einem Kopf (50) versehen ist, **dadurch gekennzeichnet, dass** es folgende Schritte umfasst:
- Auftragen der Normalen an der Oberfläche an mehreren Punkten entlang der Spirale (210),
- den Kopf (50) des Werkzeugs (5) der Spirale folgen lassen, indem der Kopf (5) so ausgerichtet wird, dass er der Ausrichtung der vorgegebenen Normalen folgt.

2. Bearbeitungsverfahren zum Erzielen einer Verzahnung an der Oberfläche einer beliebigen Form nach Anspruch 1, **dadurch gekennzeichnet, dass** die Bearbeitung mit einem Werkzeug (5) durchgeführt wird, das einen Kopf (50) mit einer abgeschrägten Form aufweist.

3. Bearbeitungsverfahren zum Erzielen einer Verzahnung an der Oberfläche einer beliebigen Form nach Anspruch 2, **dadurch gekennzeichnet, dass** die Bearbeitung entlang einer Spirale (210) mit geringer Neigung so durchgeführt wird, dass eine Verzahnung des Typs Geradverzahnung erhalten wird.

4. Bearbeitungsverfahren zum Erzielen einer Verzahnung an der Oberfläche einer beliebigen Form nach Anspruch 2, **dadurch gekennzeichnet, dass** die Bearbeitung entlang mehreren Spiralen unterschiedlicher Neigung mit jeweils unterschiedlichen Tiefen durchgeführt wird, so dass eine Verzahnung mit Spanbrecher erhalten wird.

5. Bearbeitungsverfahren zum Erzielen einer Verzahnung an der Oberfläche einer beliebigen Form nach Anspruch 2, **dadurch gekennzeichnet, dass** die Bearbeitung entlang zwei Spiralen (211, 212) mit entgegengesetzten Steigungen durchgeführt wird, so dass Zähne (214) erhalten werden, wobei die Zähne (214) die Zwischenräume als Basis (213) haben, die durch die Überschneidungen der beiden Spiralen (211, 212) gebildet sind.

6. Bearbeitungsverfahren zum Erzielen einer Verzahnung an der Oberfläche einer beliebigen Form nach Anspruch 5, **dadurch gekennzeichnet, dass** die Bearbeitung entlang den beiden Spiralen (211, 212) mit gleicher Tiefe durchgeführt wird, so dass eine Verzahnung des Typs Diamantspitze erhalten wird, wobei die Zähne senkrecht zur Hülle ausgerichtet sind.

7. Bearbeitungsverfahren zum Erzielen einer Verzahnung an der Oberfläche einer beliebigen Form nach Anspruch 6, **dadurch gekennzeichnet, dass** die Steigungen der beiden Spiralen (211, 212) konstant und gleich sind, so dass Zähne (214) erhalten werden, die die Form von regelmäßigen Pyramiden (215) aufweisen.

8. Bearbeitungsverfahren zum Erzielen einer Verzahnung an der Oberfläche einer beliebigen Form nach einem der Ansprüche 5 bis 7, **dadurch gekennzeichnet, dass** die beiden Spiralen (212, 213) unterschiedliche Neigungen haben, so dass die durch den Weg des Werkzeugs (5) ausgebildeten Zähne (214) nicht in Reihen angeordnet sind.

9. Bearbeitungsverfahren zum Erzielen einer Verzahnung an der Oberfläche einer beliebigen Form nach einem der Ansprüche 1 bis 6 und 8, **dadurch gekennzeichnet, dass** die Steigung jeder Spirale (210, 211, 212) entlang der Hülle der beliebigen Form konstant ist.

10. Bearbeitungsverfahren zum Erzielen einer Verzahnung an der Oberfläche einer beliebigen Form nach Anspruch 5, **dadurch gekennzeichnet, dass** die Zähne (214) an ihrem oberen Abschnitt flach oder strahlenförmig sein können, indem ein ebener oder strahlenförmiger Abschnitt in das Profil des abgeschrägten Kopfes (50) des Werkzeugs (5) eingebracht wird.

11. Verwendung des Verfahrens nach den Ansprüchen 1 bis 10 zur Bildung einer Raspel (1), die den intramedullären Kanal eines Knochens präparieren soll, damit dieser eine Prothese aufnimmt, **dadurch gekennzeichnet, dass** die Raspel (1) einen Körper (2) mit variablem Querschnitt aufweist, der entlang einer im Wesentlichen parallel zur Längsachse des Kanals verlaufenden Achse (3) langgestreckt ist, durch eine sogenannte obere Fläche (20) und eine Außenhülle (21) gebildet ist und eine Form besitzt, die mit der Innenwand des intramedullären Kanals des Knochens zusammenwirken kann.

12. Verwendung des Verfahrens nach Anspruch 11, **dadurch gekennzeichnet, dass** der Körper (2) der Raspel (1) in einer profilierten Form (22) endet, so dass das Einführen der Raspel (1) in den Kanal vereinfacht ist.

13. Verwendung des Verfahrens nach Anspruch 11 oder 12, **dadurch gekennzeichnet, dass** geometrische Steck- oder Buchsenelemente, die an der oberen Fläche (20) des Körpers (2) angeordnet sind, ein Einrastsystem bilden, das ein Ansatzstück aufnehmen soll, welches das Ende eines Stiels zur Handhabung der Raspel (1) im Kanal bildet und gegebenenfalls prothetische Probebauteile aufnehmen soll.

14. Verwendung des Verfahrens nach einem der Ansprüche 11 bis 13, **dadurch gekennzeichnet, dass** die obere Fläche (20) des Körpers (2) eben ist oder einen Zapfen aufweist, der das Anschweißen eines Stiels zur Handhabung der Raspel (1) im Knochen ermöglicht.

## Claims

1. Method of machining in order to produce teeth on the surface of an unspecified shape by a tool (5) provided with a head (50), along at least one geometric curve traced on the casing of the unspecified shape and having the shape of a helix (210), **characterised in that** it comprises the following pitches:
- tracing the normal to the surface at a plurality of points taken along the helix (210),
- causing the head (50) of the tool (5) to follow the helix, by directing the head (5) so that it follows the orientation of the predetermined normals.

2. Method of machining in order to produce teeth on the surface of an unspecified shape according to Claim 1, **characterised in that** the machining is carried out with a tool (5) having a head (50) of bevelled shape.

3. Method of machining in order to produce teeth on the surface of an unspecified shape according to Claim 2, **characterised in that** the machining is carried out along a helix (210) with a shallow gradient, so as to produce teeth having a straight teeth-type appearance.

4. Method of machining in order to produce teeth on the surface of an unspecified shape according to Claim 2, **characterised in that** the machining is carried out at different depths along several helices with different gradients respectively, so as to produce teeth with chip breakers.

5. Method of machining in order to produce teeth on the surface of an unspecified shape according to Claim 2, **characterised in that** the machining is carried out along two helices (211, 212) with inverted pitch, so as to produce teeth (214), the teeth (214) having as their base (213) the spaces formed by the intersections of the two helices (211, 212).

6. Method of machining in order to produce teeth on the surface of an unspecified shape according to Claim 5, **characterised in that** the machining is carried out along the two helices (211, 212) at the same depth, so as to produce diamond tip-type teeth, the orientation of the teeth being normal to the casing.

7. Method of machining in order to produce teeth on the surface of an unspecified shape according to Claim 6, **characterised in that** the pitches of the two helices (211, 212) are constant and equal, so as to produce teeth (214) having the shape of regular pyramids (215).

8. Method of machining in order to produce teeth on the surface of an unspecified shape according to any one of Claims 5 to 7, **characterised in that** the two helices (212, 213) have different gradients so that the teeth (214) formed by the passage of the tool (5) are not aligned.

9. Method of machining in order to produce teeth on the surface of an unspecified shape according to any one of Claims 1 to 6 and 8, **characterised in that** the pitch of each helix (210, 211, 212) is constant along the casing of the unspecified shape.

10. Method of machining in order to produce teeth on the surface of an unspecified shape according to Claim 5, **characterised in that** the teeth (214) can be flat or radiating over their upper part, by including in the profile of the bevelled head (50) of the tool (5) a plane or radiating portion.

11. Use of the method according to Claims 1 to 10 in order to form a rasp (1) intended to prepare the intramedullary canal of a bone to receive a prosthesis, **characterised in that** the rasp (1) comprises a body (2), extended along an axis (3) substantially parallel to the longitudinal axis of the canal, with variable cross section, constituted by a face known as the upper face (20) and an outer casing (21), and has a shape capable of collaborating with the inner wall of the intramedullary canal of the bone.

12. Use of the method according to Claim 11, **characterised in that** the body (2) of the rasp (1) ends in a profiled shape (22) in order to make it easier to drive the rasp (1) into the canal.

13. Use of the method according to Claim 11 or 12, **characterised in that** male or female geometric elements, arranged on the upper face (20) of the body (2) constitute a snap-on system intended to receive an endpiece constituting the end of a handle for manipulating the rasp (1) in the canal and intended to receive trial prosthetic components, if appropriate.

14. Use of the method according to any one of Claims 11 to 13, **characterised in that** the upper face (20) of the body (2) is plane or comprises a tenon allowing a handle to be welded on in order to manipulate the rasp (1) in the bone.
